# EUROPEAN PATENT SPECIFICATION

(11) **EP 0 894 493 B1**
(45) Date of publication and mention of the grant of the patent: **27.12.2006**
(21) Application number: 97305722.7
(22) Date of filing: 30.07.1997
(51) Int. Cl.: A61K 8/64, A61Q 19/00, A61Q 19/04

(54) **Pigmentary agent**
Pigmentstoff
Matière pigmentaire

(43) Date of publication of application: 03.02.1999
(73) Proprietor: Ramaiah, Abburi, Prof., Nagar, New Delhi 110 016 (IN)
(72) Inventor: Ramaiah, Abburi, Prof., Nagar, New Delhi 110 016 (IN)
(74) Representative: Simpson, Alison Elizabeth Fraser

(56) References cited:
- EP-A- 0 246 753
- EP-A- 0 497 341
- WO-A-89/04209
- WO-A-91/07982
- WO-A-92/13958
- WO-A-95/08630
- US-A- 5 252 718

## Description

### FIELD OF THE INVENTION

This invention relates to a pigmentary agent which may be used for treatment of pigmentary disorders such as vitiligo. The preparation may also be advantageously employed as a cosmetic preparation for tanning of skin. In either instance, an exposure of the skin surface to sunlight is not necessary to cause a tanning of the skin surface. This invention also relates to a vehicle for peptides having a molecular weight of upto 1500.

### PRIOR ART

It is generally known that melanocytic cells are responsible for pigmentation of the skin. Thus, a greater number of melanocytes present per/cm² of skin surface area causes a greater pigmentation. Vitiligo is a disorder caused by a deficiency or absence of functional melanocyte cells in the dermal-epidermal junction.

Preparations for treatment of vitiligo are known in the art. but which were not satisfactory. Such preparations were taken orally or applied to the surface of the skin of a patient. Another treatment known in the art is the Psoralens and UVA therapy. However, such known methods required a prolonged treatement as long as one to many years. In addition, Psoralen are toxic to liver and carcinogenic. Moreover, such known treatments were more often found to be ineffective.

Reference is now made to such sun screens or cosmetic preparation used for protecting the skin. Exposure to sunlight reults in tanning of skin. However, the ultra violet rays present in sunlight causes skin cancer. Thus, the sun screens known in the art were applied to prevent a penetration of the ultra violet rays.

### OBJECTS OF THE INVENTION

An object of this invention is to propose a cosmetic or tanning preparation which can advantageously be employed to a skin surface without the necessity of an exposure to sunlight.

Another object of this invention is to propose a pigmetary agent for treatment of pigmentation disorders and which requires only an application to the affected skin and without the necessity of exposure to sunlight.

Yet another object of this invention is to propose a tanning preparation of pigmentary agent which requires a small concentration of the active ingredient.

A further object of this invention is to propose a tanning preparation or pigmentary agent which does not poduce any hamful effects.

Yet a further object of this invention is to proposes vehicle for an active agent for a pigmentary or tanning preparation and as well as a carrier for other peptides having a molecular weight of upto 1500.

### DESCRIPTION OF THE INVENTION

According to this invention there is provided a composition for treatment of pigmentation disorders and tanning applications on a skin surface without the necessity of exposure to sunlight comprising 10-50% of solvent mixed with 10-40% of glycols, 10-40% of a penetration enhancing agent and 0.02% - 0.5% of selective active fragments of basic fibroblast growth factor (bFGF) mixed therein.

Further, according to this invention there is provided a process for the preparation of a composition compising mixing said solvent with said glycol at ambient temperature and pressure, adding penetration enhancing agent therein .

The present invention resides in a first aspect of a vehicle for the active agent. and a second aspect of selecting an active agent which was not hitherto known as a tanning agent. which when applied to a skin surface causes a pigmentation without an exposure to sunlight. Thus, it has now been found that the presence of the active agent in the vehicle stimulates the proliferation of melanocytic cells and thus cause a tanning of the skin surface. It ha also been found that such an active agent in the particular vehicle causes a satisfactory pigmentation of the skin surface without exposure to sunlight.

In accordance with this invention a vehicle is prepared by mixing 10-50% weight by weight of solvent mixed with 10-40% of glycols and 10-40% weight by weight of penetration enhancing agent. at an ambient temperatue, preferably at a temperature around 25°C.to to this vehicle, 0.2% to 0.5% weight by weight of selective active fragment of basic fibroblast growth factor (bFGF) is added and mixed thoroughly therewith to obtain the composition of the present invention. The solvent suitable for the present composition compries alcohols selected from isopropyl alcohol, propyl alcohol or ethyl alcohol. The glycol comprises propylene glycol or polyethylene glycol. The penetration enhancing agents comprises propyl, iopropyl or ethyl myristate or propyl or ethyl stearate or propyl, isopropyl or ethyl palmitate.

The composition used for treating vitiligo and for tanning of white skin according to a preferred embodiment is herein described and illustrated in the following examples.

### EXAMPLE 1

Effect of topical application of 106-115 of bFGF in various formulations at 0.02% on repigmenting the depigmented quinea pig ear lobes.

The controls are the car lobe topically applied with the formulations. These are not different from the spontaneously repigmenting ear lobes. There was no difference in the speed of repigmentation irrespective of the formulation applied. The total number of control are 12 and the average extent of pigmentation at the end of 48 days is 35%. The effect of topical application of the above fragment in various formulations is evaluated for statistical significance using wilcoxan rank sum test.

**TABLE 1**

| COMPOSITION: | ANIMAL NO. | % PIGMENTATION AFTER 48 DAYS |
|---|---|---|
| 50% isopopyl | 48 | 95 |
| alcohol | | |
| + | | |
| 40% propylene | 49 | 80 |
| glycol | | |
| + | | |
| 10% isopropyl | 50 | 95 |
| myristate and | | |
| + | | |
| 0.02% active fragment by bFGF | | |

The selective active fragment of basic fibroblast growth factor (bFGF) used for preparation of the solution:
1. bFGF 106-115
and its modified forms.

Reference is now made to the active fragment, which is a peptide. The biological function is not only important in its open structure but also the 3 dimensional folding of the protein. If a protein is injected in its linear form, it would have no biological function. Thus, the protein should have a specific shape so that it is recognized by the responding cell. A molecule of substantial length cannot be applied by means of a local treatment as it cannot penetrate the stratum corneum of the skin; therefore a fragmentation of native protein is necessary but simultaneously the fragment should be small enough so that it is effective and having the capability of penetration. The fragments identified hereabove are effective in pigmenting the dipigmented skin.

In accordance with this invention, the melanocytic cell in the vitiligo patch are either absent or not functional melanocytes. Thus, the melanocytes in the perilessional area or in the outer sheath of hair follicle migrate to the vitiligo patch and start to function.

Reference is now made to the vehicle for the active agent. The description hereabove is with reference to a vehicle having a particular active agent for tanning application. However, the vehicle can advantageously be employed for other peptides as an active agent.

Peptides are known in the art which are adminstered to a patient. However, such peptides could not hitherto be administrated orally or topically and need to be administered only in the form of an injection. The advantage of the present vehicle is that the peptide having a molecular weight of upto 1500 can now be administered topically, and which was not hitherto possible.

Reference is made to peptides having a molecular weight of upto 1500. Though studies have yet to be effected, it is believed that the vehicle of the present invention can also be employed for topical administration of peptides having a molecular weight of more tha than 1500.

Accordingly, and as by way of exemplary embodiments the other peptides that can be employed are:
a) melanotropins for stimulation of melanognesis/increased proliferation of melanocytes
b) granulocyte colony stimulating factor which stermulates the defence mechanism of the body
c) fusion peptides
d. interleukins.

### DESCRIPTION OF INVENTION WITH RESPECT TO ACCOMPANYING DRAWINGS:

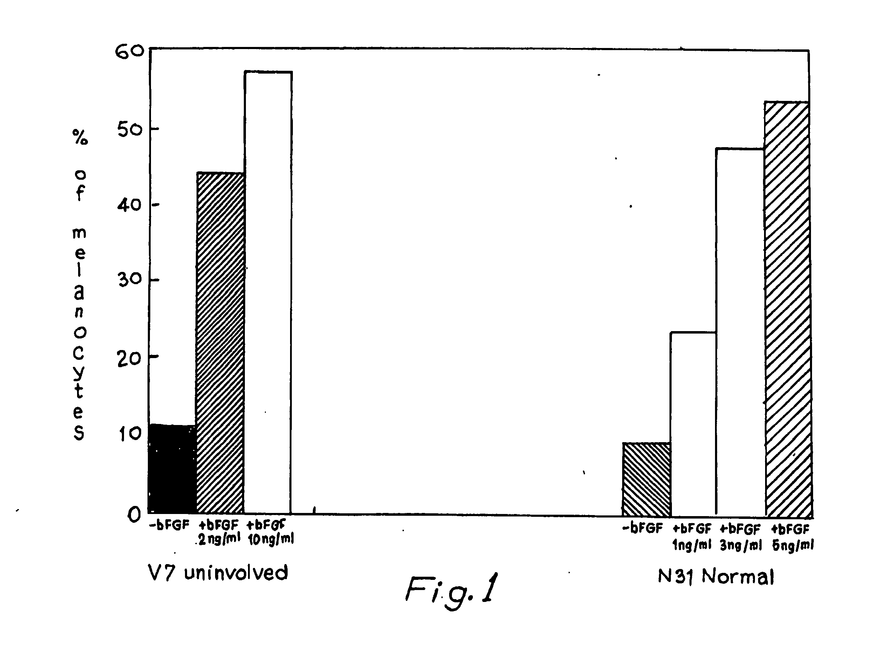

- Fig.1: shows percentage of melanocytes from an uninvolved area of a vitiligo patient V7 in response to different concentrations of bFGF compared to a normal person.

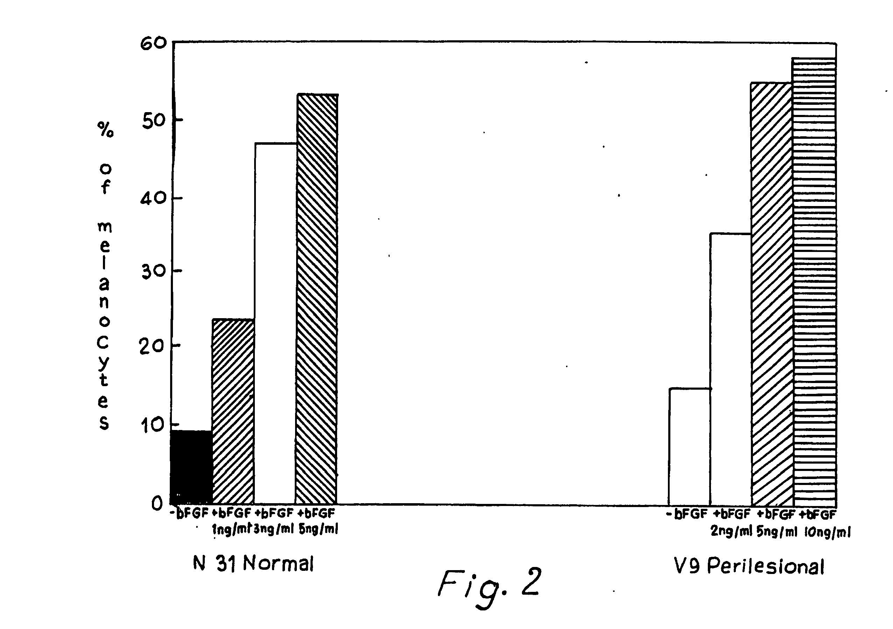

- Fig.2: shows percentage of melaanocytes from the perilesional areas of a vitiligo subject V9 in response to different concentrations of bFGF compared to a normal person.

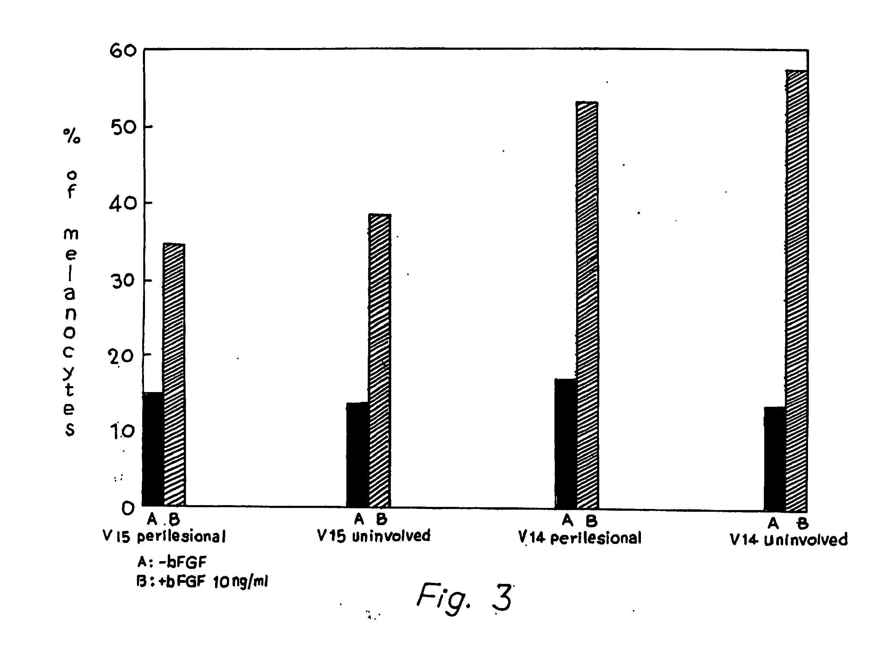

- Fig.3: shows effect of bFGF on % melanocytes in mixed cultures from the uninvolved and perilesional areas of vitiligo subjects.

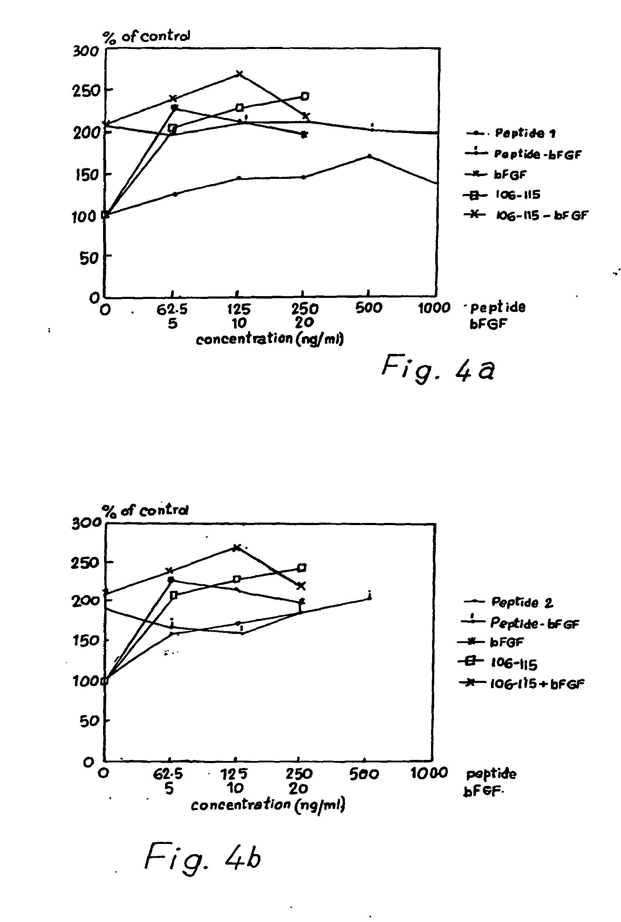

- Fig.4(a): shows the effect of fragment of 6FGF (106-115) on the melanocyte prolifeation.
- Fig.4(b): shows the effect of active fragment of bFGF of peptide referred in Table 2 hereinbelow on the proliferation of melanocyte.

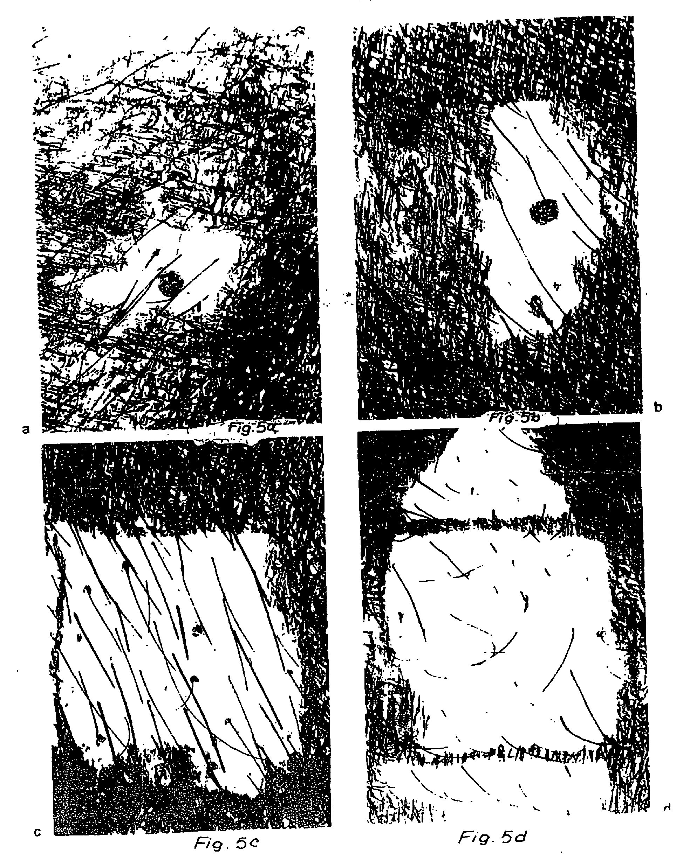

- Fig.5(a): to (d): shows the effect of topical application of active fragment bFGF (106-115) at different concentration of 1% (Fig.5(a), 0.5% (Fig.5b), 0.25% (Fig.5c) and without any active fragment (Fig.d) on the pigmentation of skin of a yucatan swine.

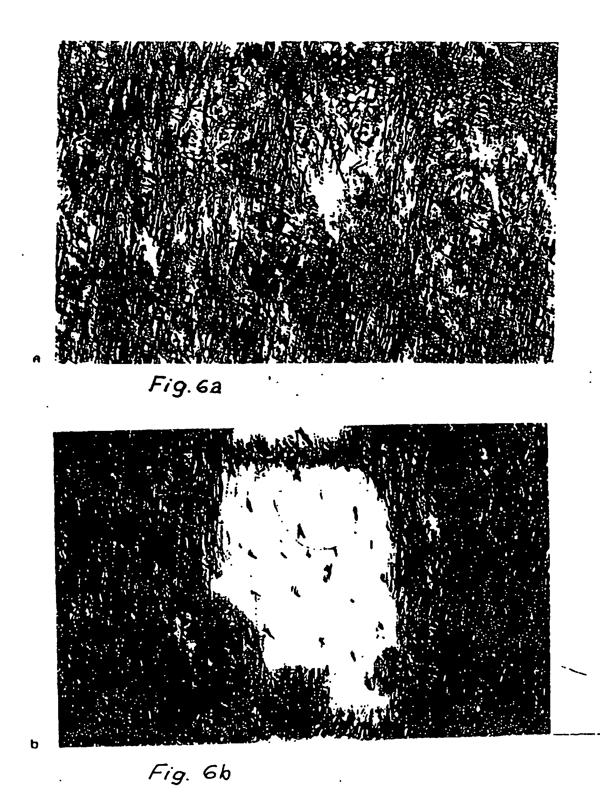

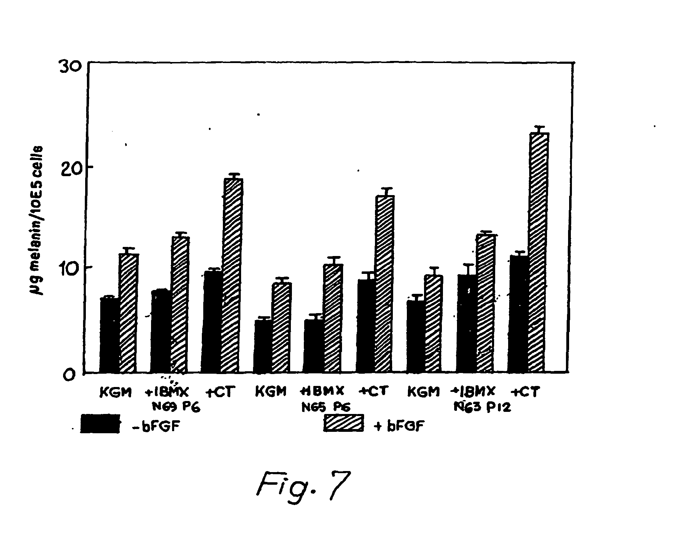

- Fig.6A 6B: show the effect of topical application of active fragment bFGF (106-115) applied for 6 weeks and the result then observed after 20 weeks (Fig.6A) on the extent of pigmentation of the skin of a Yucatan swine and Fig.B is without the fragment and Fig.7 shows the effect of bFGF on melanin content/cell

**TABLE 2**

| |
|---|
| Sequence of the peptides tested |
| bFGF 106-115 H- Tyr Arg Ser Arg Lys Tyr Ser Ser Trp Tyr-NH2 106 107 108 109 110 111 112 113 114 115 |
| Activation Replace Ser 112 by Glu: resembles phosphorylated Ser. |
| Peptide 1: H- Tyr Arg Ser Arg Lys Tyr Glu Ser Trp Tyr-NH2 106 107 108 109 110 111 112 113 114 115 |
| Cycilsation: Make the structure more rigid. Whole bFGF is more |
| rigid than 106-115 peptide. More rigid: better receptor binding. |
| Also results in more lipohpyllic compound. |
| Peptide 2: Cycol ( Tyr Arg Ser Arg Lys Tyr Ser Ser Trp Tyr ) 106 107 108 109 110 111 112 113 114 115 |
| Peptide 3: Cycol(Gly  Tyr  Arg  Ser  Arg  Lys  Tyr  Ser  Ser  Trp  Tyr ) 106  107  108  109  110  111  112  113  114  115 |
| Lipophyllicity: Make the peptides more penetrating. Petentration |
| occurs probably through the hair follicle, a lipophilic |
| environment: |
| Peptide 4: POHC H(CH) CO-Tyr Arg Ser Arg Lys Tyr Ser Ser Trp Tyr-NH2 106 107 108 109 110 111 112 113 114 115 |
| Peptide 5: Tyr Arg Ser Arg Lys Tyr Ser Ser Trp Tyr-NH(CH) C H OHp 106 107 108 109 110 111 112 113 114 115 |

## Claims

1. A preparation for carrying peptides having a molecular weight of upto 1500 comprising 10-50% of solvent, 10-40% of glycols, and 10-40% of a penetration enhancing agent.

2. A preparation for treating pigmentation disorders and tanning of skin without exposure to sun comprising a preparation according to Claim 1 and further comprising 0.02 to 0.5% of selective fragment ofbFGF 106-115, having the sequence H-Tyr-Arg-Ser-Arg-Lys-Tyr-Ser-Ser-Trp-Tyr-NH₂.

3. A preparation as claimed in Claim 2 wherein said selective active fragment of bFGF comprises bFGF 106-115, modified by cyclisation, addition of a lipophilic group or substitution of Ser 112 with Glu.

4. A preparation as claimed in Claims 1, 2 or 3 wherein said solvent comprises alcohols selected from the group consisting of isopropyl alcohol, propyl alcohol and ethyl alcohol.

5. A composition as claimed in Claims 1 to 4 wherein said glycols are selected from the group consisting of propylene glycol and polyethylene glycol.

6. A preparation as claimed in Claims 1 to 5 wherein said penetration enhancing agent is selected from one group consisting of propyl/isopropyl or ethyl myristate, propyl/isopropyl or ethyl stearate and propyl/isopropyl or ethyl palmitate.

7. A process for the preparation of a preparation as claimed in Claim 2 comprising mixing said solvent with said glycols at an ambient temperature and pressure, adding said penetration enhancing agent thereto and then adding selective active fragments of bGFG thereto and mixing thoroughly therewith.

## Patentansprüche

1. Zubereitung für das Tragen von Peptiden mit einer Molmasse von bis zu 1500, umfassend 10 - 50 % Lösungsmittel, 10 - 40 % Glykole und 10 - 40 % penetrationsverbesserndes Mittel.

2. Zubereitung für die Behandlung von Pigmentierungsstörungen und das Bräunen der Haut ohne Aussetzen der Sonne gegenüber, umfassend eine Zubereitung nach Anspruch 1 und des Weiteren umfassend 0,02 bis 0,5 % selektives Fragment von bFGF 106-115, das die Sequenz H-Tyr-Arg-Ser-Arg-Lys-Tyr-Ser-Ser-Trp-Tyr-NH₂ aufweist.

3. Zubereitung nach Anspruch 2, wobei das selektive aktive Fragment von bFGF bFGF 106-115 umfasst, das durch Cyclisieren, Zusetzen einer lipophilen Gruppe oder Substitution von Ser 112 durch Glu modifiziert worden ist.

4. Zubereitung nach Anspruch 1, 2 oder 3, wobei das Lösungsmittel Alkohole umfasst ausgewählt aus der Gruppe bestehend aus Isopropylalkohol, Propylalkohol und Ethylalkohol.

5. Zusammensetzung nach Anspruch 1 bis 4, wobei die Glykole ausgewählt werden aus der Gruppe bestehend aus Propylenglykol und Polyethylenglykol.

6. Zubereitung nach den Ansprüchen 1 bis 5, wobei das penetrationsverbessernde Mittel aus der Gruppe ausgewählt wird bestehend aus Propyl/Isopropyl- oder Ethylmyristat, Propyl/Isopropyl- oder Ethylstearat und Propyl/Isopropyl- oder Ethylpalmitat.

7. Verfahren für die Zubereitung einer Zubereitung nach Anspruch 2, umfassend das Mischen des Lösungsmittels mit den Glykolen bei Raumtemperatur und bei Umgebungsdruck, Hinzusetzen des penetrationsverbessernden Mittels und daraufhin Hinzusetzen selektiver aktiver Fragmente von bFGF und gründliches Mischen damit.

## Revendications

1. Préparation servant au transport de peptides d'un poids moléculaire allant jusqu'à 1 500, qui comprend de 10 à 50 % d'un solvant, de 10 à 40 % de glycols et de 10 à 40 % d'un agent facilitateur de la pénétration.

2. Préparation utilisée pour le traitement de troubles de la pigmentation ou le bronzage de la peau sans exposition au soleil, qui comprend une préparation selon la revendication 1 et également de 0,02 à 0,5 % d'un fragment sélectif de la portion 106-115 du bFGF (facteur de croissance fibroblastique 2) comportant la séquence H-Tyr-Arg-Ser-Arg-Lys-Tyr-Ser-Ser-Trp-Tyr-NH₂.

3. Préparation selon la revendication 2, dans laquelle ledit fragment actif sélectif du bFGF comporte la portion 106-115 du bFGF modifiée par cyclisation, addition d'un groupement lipophile ou substitution du résidu Ser en position 112 par un résidu Glu.

4. Préparation selon la revendication 1, 2 ou 3, dans laquelle ledit solvant comprend des alcools sélectionnés parmi le groupe consistant en l'alcool isopropylique, l'alcool propylique et l'alcool éthylique.

5. Composition selon les revendications 1 à 4, dans laquelle lesdits glycols sont sélectionnés parmi le groupe consistant en le propylèneglycol et le polyéthylèneglycol.

6. Préparation selon les revendications 1 à 5, dans laquelle l'agent facilitateur de la pénétration est sélectionné parmi le groupe consistant en le myristate de propyle/d'isopropyle ou d'éthyle, le stéarate de propyle/d'isopropyle ou d'éthyle et le palmitate de propyle/d'isopropyle ou d'éthyle.

7. Procédé pour l'élaboration d'une préparation selon la revendication 2, qui comprend le mélange dudit solvant et desdits glycols à température et pression ambiantes, l'addition à ce mélange dudit agent facilitateur de la pénétration puis desdits fragments actifs sélectifs du bFGF et l'homogénéisation de l'ensemble.
